# EUROPÄISCHE PATENTSCHRIFT

(11) **EP 0 424 670 B1**
(45) Veröffentlichungstag und Bekanntmachung des Hinweises auf die Patenterteilung: **22.02.1995**
(21) Anmeldenummer: 90118102.4
(22) Anmeldetag: 20.09.1990
(51) Int. Cl.: C07K 7/06, A61K 38/00

(54) **Neue ZNS-aktive Hexapeptide mit antiamnestischer Wirkung**
ZNS-active hexapeptides with antiamnestic activity
Hexapeptides à activité ZNS exerçant une action antiamnestique

(30) Priorität: 23.09.1989 DE 3931788
(43) Veröffentlichungstag der Anmeldung: 02.05.1991
(73) Patentinhaber: HOECHST AKTIENGESELLSCHAFT, 65926 Frankfurt am Main (DE)
(72) Erfinder: Jäger, Georg, Dr., D-6232 Bad Soden (DE); Gerhards, Hermann Josef, Dr., D-6238 Hofheim am Taunus (DE); Hock, Franz, Dr., D-6110 Dieburg (DE)

(56) Entgegenhaltungen:
- EP-A- 0 179 332
- DE-A- 1 954 794
- DE-A- 1 965 102
- PEPTIDES, Proc. 19th Eur. Pept. Symp., 1986 (publ. 1987), Walter de Gruyter & Co., Berlin (DE); H. GEIGER et al., Seiten 489-492#
- EUROPEAN JOURNAL OF PHARMACOLOGY, Band 130, 1986, Elsevier Science Publishers B.V.; A. BERTOLINI et al., Seiten 19-26#

## Beschreibung

ZNS-aktive Peptide mit antiamnestischer Wirkung bzw. Wirkung auf das cholinerge System sind bereits aus der EP-A 179 332 bekannt. Die dort beschriebenen Hexapeptide haben allerdings den Nachteil, daß sie sich leicht umwandeln bzw. zersetzen, also relativ instabil sind. Im Hinblick auf die Verwendung als Arzneimittel ist dies insbesondere bezüglich der Herstellung von galenischen Zubereitungen und deren Haltbarkeit problematisch.

Aufgabe vorliegender Erfindung ist es deshalb, Verbindungen mit antiamnestischer Wirkung bereitzustellen, die stabil und somit problemlos handhabbar sind und unter deren Verwendung Arzneimittel mit langer Haltbarkeit hergestellt werden können.

Diese Aufgabe wird überraschenderweise gelöst durch die Verbindung der Formel

Ac-Met(O₂)-Glu-His-Phe-D-Lys-Phe-NH-(CH₂)₈-NH₂.

sowie dessen physiologisch verträgliche Salze.

Physiologisch verträgliche Salze der obigen Verbindung sind beispielsweise solche, die basische Gruppen, also insbesondere die Aminogruppen, mit anorganischen oder organischen Säuren bilden. Solche Säuren sind beispielsweise Salzsäure, Schwefelsäure, Phosphorsäure, Essigsäure, Citronensäure, Benzoesäure, Maleinsäure, Fumarsäure, Weinsäure und p-Toluolsulfonsäure. Eine bevorzugte Säure ist Essigsäure.

Die Erfindung betrifft ferner ein Verfahren zur Herstellung der obengenannten Verbindung, das dadurch gekennzeichnet ist, daß man ein Fragment mit N-terminaler freier Aminogruppe kondensiert mit einem Fragment mit C-terminaler freier Carboxylgruppe, wobei in diesen Fragmenten an der Reaktion nicht beteiligte primäre und sekundäre Aminogruppen mit sauer abspaltbaren Schutzgruppen vom Urethantyp (wie z.B. Boc) und Carboxylgruppen mit sauer abspaltbaren Schutzgruppen vom Estertyp (wie z.B. Bu^{t}) geschützt vorliegen, in der so erhaltenen Verbindung zum Schutz von Amino- bzw. Carboxylgruppen eingeführte Schutzgruppen sauer abspaltet und die Verbindung gegebenenfalls in ihre physiologisch verträglichen Salze überführt.

Schutzgruppen vom Urethantyp und Estertyp sind in Schröder, Lübke, Academic Press, The Peptides, Bd. I, New York, London 1965, Seite 22 ff., und Seite 52 ff. sowie in A. Hubbuch, Kontakte Merck 3/79, Seiten 14-23 beschrieben.

Man führt das Verfahren vorteilhaft in der Weise durch, daß man
a) eine Verbindung der Formel IIa mit einer Verbindung der Formel IIIa kondensiert,

   Ac-Met(O)₂-OH (IIa)

   H-Glu-His-Phe-D-Lys-Phe-X (IIIa)
b) eine Verbindung der Formel IIb mit einer Verbindung der Formel IIIb kondensiert,

   Ac-Met(O₂)-Glu-OH (IIb)

   H-His-Phe-D-Lys-Phe-X (IIIb)
c) eine Verbindung der Formel IIc mit einer Verbindung der Formel IIIc kondensiert,

   Ac-Met(O₂)-Glu-His-OH (IIc)

   H-Phe-D-Lys-Phe-X (IIIc)
d) eine Verbindung der Formel IId mit einer Verbindung der Formel IIId kondensiert,

   Ac-Met(O₂)-Glu-His-Phe-OH (IId)

   H-D-Lys-Phe-X (IIId)

   oder
e) eine Verbindung der Formel IIe mit einer Verbindung der Formel IIIe kondensiert,

   Ac-Met(O₂)-Glu-His-Phe-D-Lys-OH (IIe)

   H-Phe-X (IIIe)

wobei X für geschütztes -NH(CH₂)ₙNH₂ steht, jedoch freie primäre und sekundäre Aminogruppen, die N-terminalen Gruppen der Verbindungen der Formeln IIIa-e ausgenommen, mit sauer abspaltbaren Schutzgruppen vom Urethantyp geschützt sind und freie Carboxylgruppen, die C-terminalen Gruppen der Verbindungen der Formeln IIa-e ausgenommen, mit sauer abspaltbaren Schutzgruppen vom Estertyp geschützt sind, anschließend in der nach a) - e) erhaltenen Verbindung zum Schutz von Amino- bzw. Carboxylgruppen eingeführte Schutzgruppen sauer abspaltet und die erhaltene Verbindung gegebenenfalls in ihre physiologisch verträglichen Salze überführt. Die Umsetzung der Verbindung der Formel IIa mit der Verbindung der Formel IIIa ist bevorzugt.

Die Ausgangsverbindungen der Formeln IIa-e und IIIa-e sind bekannt oder in an sich bekannter Weise beispielsweise durch Fragmentkondensation, zugänglich.

Bei der Synthese der erfindungsgemäßen Peptide kommt als N^{α}-Schutzgruppe bevorzugt der Benzyloxycarbonyl- oder 9-Fluorenylmethoxycarbonylrest in Frage, als Carboxylschutzgruppe der tert.-Butylrest.

Die Kondensation nach dem erfindungsgemäßen Verfahren erfolgt nach den allgemeinen Methoden der Peptidchemie, beispielsweise nach der Methode der gemischten Anhydride, über Aktivester, Azide oder nach der Carbodiimid-Methode, insbesondere unter Zusatz reaktionsbeschleunigender und racemisierungsverhindernder Substanzen wie z.B. 1-Hydroxybenzotriazol, N-Hydroxysuccinimid, 3-Hydroxy-4-oxo-3,4-dihydro-1,2,3-benzotriazin, N-Hydroxy-5-norbornen-2,3-dicarboximid, ferner unter Verwendung aktivierter Derivate des 1-Hydroxybenzotriazols oder Anhydriden von Phosphor-, Phosphon- und Phosphinsäuren.

Lösungsmittel sind Dimethylformamid, Dimethylacetamid, Hexamethylphosphorsäuretriamid, N-Methylpyrrolidon oder Dimethylsulfoxid. Sofern die Löslichkeit der Komponenten es erlaubt, können auch Lösungsmittel wie Methylenchlorid oder Chloroform eingesetzt werden. Die genannten Methoden sind z.B. in Meienhofer-Gross: "The Peptides", Academic Press, Vol. I. (1979) beschrieben.

Die erfindungsgemäße Verbindung ist aufgrund ihrer pharmakologischen Eigenschaften für die Behandlung cognitiver Dysfunktionen unterschiedlicher Genese, wie sie z.B. bei der Alzheimer'schen Krankheit oder der senilen Demenz auftreten, geeignet. Die Verbesserung cognitiver Funktionen durch die erfindungsgemäßen Verbindungen (nootrope Wirkung) wurde im Tierexperiment an Mäusen mittels des inhibitory (passive) avoidance-Tests (step-through-Modell) und an Ratten mittels des radial-maze-Tests (8-armiger radialer Irrgarten) geprüft. Es handelt sich hierbei um literaturbeschriebene Standardtests zur Prüfung von Lern-und Gedächtnisfunktionen im Tierexperiment. (Inhibitory avoidance J. Bures, O. Buresova und J. Huston in "Techniques and Basic Experiments for the Study of Brain and Behavior", Elsevier Scientific Publishers, Amsterdam 1983;
Radial Maze: U. Stäubli, M. Baudry und G. Lynch, Behavioral and Neural Biology 40, 58-69 (1984).)

Entsprechend den genannten Literaturangaben wird eine Substanz dann als nootrop wirksam bezeichnet, wenn sie bei den Versuchstieren der durch Scopolamin, Elektroschock oder Inhibitoren der Proteinsynthese induzierte Erhöhung der Fehlerrate in diesen Tests entgegenwirkt oder sie gar aufzuheben vermag.

Die Versuche wurden nach den obenbeschriebenen Testverfahren durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen zeigte sich darin, daß die Scopolamin-induzierte Amnesie im inhibitory-avoidance-Test sich mit einer MED (minimal effektive Dosis) von 0,3 - 1 »g/kg s.c. und im radial-maze-Test mit einer MED von 10 »g/kg s.c. aufheben läßt, während die Vergleichsverbindung in diesen Tests eine MED von 500 - 1000 mg/kg p.o. besitzt.

Beim Menschen wirkt das erfindungsgemäße Peptid stimmungsaufhellend, antidepressiv und anxiolytisch. Es erhöht die Aufmerksamkeit gegenüber der Umwelt, verbessert die Lern- und Gedächtnisleistung, wirkt sich günstig bei Resozialisierungsprozessen aus und kann bei allen Erkrankungen, post-traumatischen und degenerativen Hirnschäden angewandt werden, die mit verminderter Funktion des zentralen Acetylcholinstoffwechsels verbunden sind, z.B. milder Dementia und auch Frühformen der Alzheimer'schen Krankheit etc. Weiterhin vermindert es die Hyperemotionalität.

Die Erfindung betrifft daher auch pharmazeutische Präparate, die diese obengenannte Verbindung enthalten.

Bei einem normalgewichtigen Erwachsenen wird die intranasale Applikation vorzugsweise in einer Dosierung von 0,1 »g bis 1 mg pro Dosis angewandt, besonders bevorzugt sind 1 bis 500, insbesondere 5 bis 200 »g pro Dosis. Die erfindungsgemäßen Arzneimittel können beispielsweise bis zu 6mal, vorzugsweise bis zu 3mal pro Tag appliziert werden. In vielen Fällen genügt auch die Applikation einer Dosis pro Tag. Die erfindungsgemäße Verbindung kann auch subcutan mit 0,001 bis 10 »g/kg, vorzugsweise 0,01 bis 5 »g/kg, insbesondere 0,05 bis 2 »g/kg verabreicht werden. In Abhängigkeit von der Konstitution ist sie mehr oder weniger peroral resorbierbar. Der Dosisspielraum bei peroraler Gabe ist vergleichsweise groß und liegt zwischen 0,1 und 50 mg täglich, auf mehrere Verabreichungen verteilt.

Bevorzugte Einzeldosis ist bei der obengenannten Verbindung 0,1 bis 10 mg.

Pharmazeutische Präparate enthalten eine wirksame Menge eines Wirkstoffs der obengenannten Verbindung zusammen mit einem anorganischen oder organischen pharmazeutisch verwendbaren Trägerstoff. Die Anwendung kann intranasal, intravenös, subcutan oder peroral erfolgen.

Für eine orale Anwendungsform wird die aktive Verbindung mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige oder alkoholische Lösungen. Als inerte Träger können z.B. Gummi arabicum, Magnesiumcarbonat, Kaliumphosphat, Milchzucker, Glucose oder Stärke, insbesondere Maisstärke, verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation wird die aktive Verbindung oder dessen physiologisch verträgliche Salze, gewünschtenfalls mit den dafür üblichen Substanzen wie Lösungsvermittler, Emulgatoren oder weitere Hilfsstoffe in Lösung, Suspension oder Emulsion gebracht. Als Lösungsmittel für die neue aktive Verbindung und die entsprechenden physiologisch verträglichen Salze kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

### Beispiel 1

### Ac-Met(O₂)-Glu-His-Phe-D-Lys-Phe-NH-(CH₂)₈-NH₂ · 2 CH₃COOH

a) Acetyl-L-methioninsulfon (Ac-Met(O₂)-OH)
   10,0 g Acetyl-L-methionin (Fluka) werden in 50 ml Trifluoressigsäure gelöst und unter Rühren bei -20°C mit der Mischung aus 12,5 ml 30 %igem Wasserstoffperoxid und 12,5 ml Wasser umgesetzt. Nach Entfernung der Kühlung wird bei Raumtemperatur nachgerührt, mit 1 g Palladium/Kohle-Katalysator versetzt, 5 Minuten gerührt und vom Katalysator abgesaugt. Das Filtrat wird im Vakuum eingedampft, der ölige Rückstand mit 11 Diisopropylether behandelt und die erhaltene feste Substanz abfiltriert. Das mit Diisopropylether gewaschene Produkt wird im Vakuum getrocknet.
   Ausbeute 10,31 g (88 % d.Th.)
   Schmp. 141 - 146°C
   [α]_{D} : +17,6° (C = 1 in Methanol)
   + 5,1° (C = 1 in Wasser)
b) Ac-Met(O₂)-Glu(OtBu)-His-Phe-D-Lys(Boc)-Phe-NH-(CH₂)₈-NH-Boc
   32,1 g H-Glu(OtBu)-His-Phe-D-Lys(Boc)-Phe-NH-(CH₂)₈-NH-Boc · HTosOH (Beispiel 11a der EP-A 179 332) werden in 250 ml Dimethylformamid gelöst, 5,86 ml N-Ethylmorpholin, 3,44 g 1-Hydroxybenzotriazol und 5,7 g Ac-Met(O₂)-OH zugegeben und nach dem Abkühlen auf 0°C mit 5,25 g Dicyclohexylcarbodiimid versetzt. Die Reaktionsmischung wird 15 Stunden bei Raumtemperatur gerührt, vom ausgefallenen Dicyclohexylharnstoff abfiltriert, mit Dimethylformamid nachgewaschen und das Filtrat im Vakuum eingedampft. Der ölige Rückstand wird in wassergesättigtem n-Butanol gelöst, die Lösung nacheinander mit wäßriger Kaliumhydrogensulfat- und Natriumhydrogencarbonat-Lösung sowie Wasser extrahiert. Die abgetrennte n-Butanolphase wird im Vakuum eingedampft und der ölige Rückstand in warmem Methanol gelöst. Die Lösung wird in 1 %ige wäßrige Natriumhydrogencarbonat-Lösung gegossen, nachgerührt und das ausgefallene Produkt abfiltriert, mit Wasser gewaschen und im Vakuum getrocknet.
   Das getrocknete Produkt wird fünfmal hintereinander in einem Gemisch aus n-Heptan-n-Butanol-Methanol 416:45:413 unter Verwendung von ^{(R)}Dicalite als Filterhilfsmittel umgelöst. Nach dem letzten Abfiltrieren wird der Rückstand mit Methanol versetzt, nach Rühren vom Filterhilfsmittel abfiltriert, das Filtrat im Vakuum eindestilliert und der Rückstand mit Wasser verrührt. Das Produkt wird abfiltriert, mit Wasser gewaschen und im Vakuum getrocknet.
   Ausbeute 14,5 g (44 % d.Th.).
c) Ac-Met(O₂)-Glu-His-Phe-D-Lys-Phe-NH(CH₂)₈-NH₂ · 2 CH₃COOH
   10,2 g Ac-Met(O₂)-Glu(OtBu)-His-Phe-D-Lys(Boc)-Phe-NH-(CH₂)₈-Boc werden in der Mischung aus 40,8 ml Trifluoressigsäure und 4,5 ml Wasser gelöst. Die Lösung wird 30 Minuten gerührt und dann im Vakuum eindestilliert. Der ölige Rückstand wird in Wasser gelöst, auf eine mit ^{(R)}Amberlite IRA 93 (Acetatform) gefüllte Säule gegeben und mit Wasser eluiert. Das Eluat wird gefriergetrocknet.
   Ausbeute 9,12 g (99,9 % d.Th.).

### Vergleichsbeispiel

a) 200 mg Ac-Met(O₂)-Glu-His-Phe-D-Lys-Phe-NH(CH₂)₈-NH₂ · 2 CH₃COOH wurden in 20 ml Wasser gelöst und 8 Tage auf 80°C erhitzt. Nach dem Abkühlen wurde gefriergetrocknet. Eine HPLC-Untersuchung ergibt, daß keine Zersetzung stattgefunden hat.
   HPLC-Bedingungen:
   - Säule:: Nucleosil 120 5» C₁₈
   - Fluss:: 1,5 ml/min
   - Temperatur:: RT
   - Detektion:: Absorption bei 220 nm
   - Mobile Phase:: 800 Wasser/100 Acetonitril/3H₃PO₄ 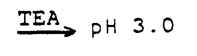
b) Eine Wiederholung von a) mit 200 mg H-Met(O₂)-Glu-His-Phe-D-Lys-Phe-NH-(CH₂)₈-NH₂ (Beispiel 16 der EP-A 179 332) ergibt eine starke Zersetzung des Substrats.

## Patentansprüche (Patentansprüche für folgende(n) Vertragsstaat(en): AT, BE, CH, DE, DK, FR, GB, IT, LI, LU, NL, SE)

1. Hexapeptid der Formel AC-Met(O₂)-Glu-His-Phe-D-Lys-Phe-NH-(CH₂)₈-NH₂, sowie dessen physiologisch verträglichen Salze.

2. Verfahren zur Herstellung eines Hexapeptids der Formel gemäß Anspruch 1, dadurch gekennzeichnet, daß man ein Fragment mit N-terminaler freier Aminogruppe kondensiert mit einem Fragment mit C-terminaler freier Carboxylgruppe, wobei in diesen Fragmenten an der Reaktion nicht beteiligte primäre und sekundäre Aminogruppen mit sauer abspaltbaren Schutzgruppen vom Urethantyp (wie z.B. Boc) und Carboxylgruppen mit sauer abspaltbaren Schutzgruppen vom Estertyp (wie z.B. Bu^{t}) geschützt vorliegen, in den so erhaltenen Verbindungen zum Schutz von Amino- bzw. Carboxylgruppen eingeführte Schutzgruppen sauer abspaltet und die Verbindungen gegebenenfalls in ihre physiologisch verträglichen Salze überführt.

3. Pharmazeutisches Mittel enthaltend ein Hexapeptid der Formel gemäß Anspruch 1.

## Patentansprüche (Patentansprüche für folgende(n) Vertragsstaat(en): ES, GR)

1. Verfahren zur Herstellung eines Hexapeptids der Formel
Ac-Met(O₂)-Glu-His-Phe-D-Lys-Phe-NH-(CH₂)₈-NH₂,
sowie dessen physiologisch verträglichen Salze, dadurch gekennzeichnet, daß man ein Fragment mit N-terminaler freier Aminogruppe kondensiert mit einem Fragment mit C-terminaler freier Carboxylgruppe, wobei in diesen Fragmenten an der Reaktion nicht beteiligte primäre und sekundäre Aminogruppen mit sauer abspaltbaren Schutzgruppen vom Urethantyp (wie z.B. Boc) und Carboxylgruppen mit sauer abspaltbaren Schutzgruppen vom Estertyp (wie z.B. Bu^{t}) geschützt vorliegen, in den so erhaltenen Verbindungen zum Schutz von Amino- bzw. Carboxylgruppen eingeführte Schutzgruppen sauer abspaltet und die Verbindungen gegebenenfalls in ihre physiologisch verträglichen Salze überführt.

2. Verfahren zur Herstellung eines pharmazeutischen Mittels, enthaltend ein Hexapeptid der Formel gemäß Anspruch 1, dadurch gekennzeichnet, daß man dieses und einen Träger in eine geeignete Darreichungsform bringt.

## Claims (Claims for the following Contracting State(s): AT, BE, CH, DE, DK, FR, GB, IT, LI, LU, NL, SE)

1. A hexapeptide of the formula Ac-Met(O₂)-Glu-His-Phe-D-Lys-Phe-NH-(CH₂)₈-NH₂ and the physiologically tolerated salts thereof.

2. A process for the preparation of a hexapeptide of the formula as claimed in claim 1, which comprises condensing a fragment with an N-terminal free amino group with a fragment with a C-terminal free carboxyl group, where in these fragments primary and secondary amino groups which are not involved in the reaction are protected with urethane-type protective groups which can be eliminated with acid (such as, for example, Boc) and carboxyl groups are protected with ester-type protective groups which can be eliminated with acid (such as, for example, Bu^{t}), eliminating with acid the protective groups introduced to protect amino and carboxyl groups in the compounds obtained in this way, and converting the compounds where appropriate into their physiologically tolerated salts.

3. A pharmaceutical composition containing a hexapeptide of the formula as claimed in claim 1.

## Claims (Claims for the following Contracting State(s): ES, GR)

1. A process for the preparation of a hexapeptide of the formula Ac-Met(O₂)-Glu-His-Phe-D-Lys-Phe-NH-(CH₂)₈-NH₂ and the physiologically tolerated salts thereof, which comprises condensing a fragment with an N-terminal free amino group with a fragment with a C-terminal free carboxyl group, where in these fragments primary and secondary amino groups which are not involved in the reaction are protected with urethane-type protective groups which can be eliminated with acid (such as, for example, Boc) and carboxyl groups are protected with ester-type protective groups which can be eliminated with acid (such as, for example, Bu^{t}), eliminating with acid the protective groups introduced to protect amino and carboxyl groups in the compounds obtained in this way, and converting the compounds where appropriate into their physiologically tolerated-salts.

2. A process for producing a pharmaceutical composition containing a hexapeptide of the formula as claimed in claim 1, which comprises converting the latter and a vehicle into a suitable administration form.

## Revendications (Revendications pour l'(les) Etat(s) contractant(s) suivant(s): AT, BE, CH, DE, DK, FR, GB, IT, LI, LU, NL, SE)

1. Hexapeptide de formule
Ac-Met(O₂)-Glu-His-Phe-D-Lys-Phe-NH-(CH₂)₈-NH₂
et sels physiologiquement acceptables de celui-ci.

2. Procédé pour la préparation d'un hexapeptide de formule selon la revendication 1, caractérisé en ce que l'on condense un fragment à groupe amino N-terminal libre avec un fragment à groupe carboxy C-terminal libre, des groupes amino primaires et secondaires présents dans ces fragments et-ne participant pas à la réaction se trouvant protégés par des groupes protecteurs du type uréthanne (comme par exemple Boc) qui peuvent être éliminés en milieu acide, et des groupes carboxy présents dans ces fragments et ne participant pas à la réaction se trouvant protégés par des groupes protecteurs du type ester (comme par exemple Bu^{t}) qui peuvent être éliminés en milieu acide, on élimine en milieu acide, dans le composé ainsi obtenu, les groupes protecteurs introduits pour la protection de groupes amino ou carboxy, et éventuellement on convertit le composé en ses sels physiologiquement acceptables.

3. Composition pharmaceutique contenant un hexapeptide de formule selon la revendication 1.

## Revendications (Revendications pour l'(les) Etat(s) contractant(s) suivant(s): ES, GR)

1. Procédé pour la préparation d'un hexapeptide de formule
Ac-Met(O₂)-Glu-His-Phe-D-Lys-Phe-NH-(CH₂)₈-NH₂
ainsi que de ses sels physiologiquement acceptables, caractérisé en ce que l'on condense un fragment à groupe amino N-terminal libre avec un fragment à groupe carboxy C-terminal libre, des groupes amino primaires et secondaires présents dans ces fragments et ne participant pas à la réaction se trouvant protégés par des groupes protecteurs du type uréthanne (comme par exemple Boc) qui peuvent être éliminés en milieu acide, et des groupes carboxy présents dans ces fragments et ne participant pas à la réaction se trouvant protégés par des groupes protecteurs du type ester (comme par exemple Bu^{t}) qui peuvent être éliminés en milieu acide, on élimine en milieu acide, dans le composé ainsi obtenu, les groupes protecteurs introduits pour la protection de groupes amino ou carboxy, et éventuellement on convertit le composé en ses sels physiologiquement acceptables.

2. Procédé pour la préparation d'une composition pharmaceutique contenant un hexapeptide de formule selon la revendication 1, caractérisé en ce qu'on le met, avec un véhicule, sous une présentation appropriée.
